# EUROPEAN PATENT APPLICATION

(11) **EP 1 260 210 A1**
(43) Date of publication of application: **27.11.2002**
(21) Application number: 01904469.2
(22) Date of filing: 15.02.2001
(51) Int. Cl.: A61K 7/00, A61K 31/01, A61L 9/01, A01N 27/00, A01N 45/00, C01B 31/02

(54) **SQUALANE CONTAINING ULTRA FINE PARTICLES OF BURNING RESIDUE OF CARBON AND METHOD FOR PRODUCING THE SAME**

(30) Priority: 24.02.2000 JP 2000047228; 07.02.2001 JP 2001030819
(71) Applicant: Phild Co., Ltd., Kamigyo-ku, Kyoto-city, Kyoto 602-0023 (JP)
(72) Inventor: HIRATA, Yoshihiro, Phild Co., Ltd., Karasumadori, Kamigyo-kuKyoto City, Ky (JP); UEDA, Yoshio, Phild Co., Ltd., Karasumadori, Kamigyo-ku Kyoto City (JP); TAKASE, Hiroaki, Phild Co., Ltd., Karasumadori, Kamigyo-ku Kyoto City (JP)
(74) Representative: VOSSIUS & PARTNER
(86) International application number: JP0101076
(87) International publication number: WO01062213

(57) **Abstract**

Squalane, which contains fullerene useful for a complex material with high functionality including effects on improving health, is provided. Squalane in which fullerene as ultrafine particles of carbon combusting residue are floating is produced by combusting mixed gas of oxygen and hydrogen in squalane in a vacuum and heating and combusting carbon with the combustion gas obtained.

## Description

### Technical Field

The present invention relates to a health promotion material comprising squalane containing ultrafine particles of carbon combustion residue such as fullerene.

The present invention relates to a method and a device for producing squalane in which ultrafine particles of carbon combustion residue such as fullerene float.

### Background Art

In recent developments of advanced technologies, the importance of material technology has increased and materials with high functionality particularly attract attention as next-generation technology. Under these circumstances, research and development of new materials with high functionality are actively carried out in every technological field. Given this factor, switching to materials having new excellent functionality is attempted and endeavors are made to review a variety of conventionally well known materials to make certain of their functionality.

A typical example of materials subjected to such research is charcoal or carbon, which has been accessible for a long time. Charcoal or carbon has been used for various purposes in various sectors. Particularly, use of its functionality such as deodorizing and antiseptic effects has been found useful. For example, a number of phenomena have been known such as building materials, the surface of which is carbonized by getting scorched, are not decayed and that small animals heal wounds by powder charcoal found at burned-out sites in forests. Additionally, positive effects of carbon on the human body in health and treatment aspects have been recognized. Because of the effects of the negative ions of carbon, use of carbon for health effects recently is recognized anew. Bincho-charcoal and bamboo charcoal are used for water quality improvement and cooking purposes, and commercialized toiletry products in which carbon is mixed including shampoos and soap exist.

Furthermore, a new material called fullerene recently was discovered as a carbon material and attracts attention as the third crystallization next to diamond and graphite. Fullerene is a carbon material. Typically, fullerene comprises 60 carbon atoms forming a spherical structure. It is a carbon molecule with a structure of a soccer-ball like pattern combining pentagons and hexagons and is a physically and chemically stable molecule. Synthesis of fullerene by a chemical reaction has not been reported. Producing fullerene by a physical method has become possible. A typical production method is: carbon vapor is generated by vaporizing graphite by arc discharge or laser beam irradiation; soot is formed by cooling down the carbon vapor generated; and in the formed soot, fullerene is generated. Fullerene generated in the soot is dissolved in a solvent and refined by isolating them by such refining methods as liquid column chromatography. The structure of fullerene, which is similar to a soccer ball, has been confirmed by the C₆₀ peak in a mass spectrum or crystal structure analysis by X-ray, and ¹³C NMR spectrum or infrared absorption spectrum, etc.

Because isolating fullerene has become possible and that a prospect for mass production of fullerene in physical methods has been found, a large variety of chemically and physically peculiar properties of fullerene have attracted attention, research on utilization of fullerene gains momentum, and their valuable utilization in various fields has been unfolded. For example, technologies for using fullerene as a superconductive material, semiconductor material or ferromagnetic material in the field of electronics; as non-linear optical material, a catalyst, etc. in the physical material field; and as micro lubricant, a buffer agent, etc. in the mechanical material field are being developed. Furthermore, utilization of fullerene for bioactive material, food material or medicines also has been proposed.

If listing typical technologies regarding carbon and fullerene which is used for health purposes, technologies using carbon itself include plaster board containing healthy carbon specially used for sick house syndrome (Japanese Patent Laid-open No.1999-58594), healthy bedding for promoting health during sleep (Japanese Patent Laid-open No.1997-51839), and technologies relating to fullerene include: a method for deactivating virus by oxygen in a singlet state, which is activated by fullerene, and light exposure (Japanese Patent Laid-open No.1997-322767) and a sun protection cosmetic the color of which does not appear to be obvious when applied, in which fullerene is dissolved in an oil-based component such as diester carvone and which is excellent in sun protection (Japanese Patent Laid-open No.1997-278625).

As for squalane, which is a marine life resource (an extract from deep sea shark liver oil) known in the prior art, it attracts attention as a health food product and its therapeutic effect has aroused interest. Recently, beauty effects of squalane are also receiving attention. The squalane concerned is clear and light-yellow-colored C₃₀H₅₀ unsaturated hydrocarbon liquid, the properties of which are known as follows: specific gravity of 0.8595, boiling point (2 mmHg) of 240 to 242°C, reflective index of 1.4965, iodine value of 377.0, and a solidifying point of below -70°C.

It is thought that beautiful skin is created by metabolism of skin cells of the human body being accelerated by squalane having an effect of increasing oxygen supply. Because squalane itself has a physicality very easy to degenerate, it was not adequate to apply it on skin in its stable state.

For this reason, use of squalane by saturating unsaturated carbon of squalane contained in unsaturated hydrocarbon was attempted. In other words, squalane which can be obtained by refining squalane in such methods as hydrogenation is clear and colorless C₃₀H₆₂ saturated hydrocarbon with properties: specific gravity of 0.8115, boiling point (3 mmHg) of 222 to 266°C, reflective index of 1.4515, and an iodine value of 0. Stabilized squalane by refining unsaturated squalane in the above-mentioned way, however, has remarkable effects including helping cutaneous respiration, preventing skin cells from aging by supplying oxygen to the cells and helping revitalization of skin. Additionally, because squalane is a substance with high affinity for skin and can be rubbed into skin well, it can produce well-moisturized skin. Moreover, having low viscosity and being quickly absorbed, squalane does not give a feeling of stickiness. It is appreciated that squalane has a nice and cool feeling that is hard to tell whether it was applied on the skin or not.

If listing technologies utilizing squalane for beauty and health purposes, there are a skin cosmetic comprising squalane and royal jelly for improving beauty effects (Japanese Patent Publication No. 1980-6608), a beauty scrub comprising an agent for scrubbing skin in which squalane is added to plant seed and an ethanol skin softening agent (Japanese Patent Laid-open No.1986-172809), a method for promoting health in which a liniment comprising powdered bath salt and squalane, and far-infrared rays effects are combined (Japanese Patent Laid-open No.1988-183076, Japanese Patent Laid-open No.1993-269214), a bath agent for maintaining good health of a bather in which cypress sawdust is filled in a paper pack (Japanese Patent Laid-open No.1993-112444) and known others. Although it is hoped that squalane has the above-mentioned effects, satisfactory beauty effects were not always able to be obtained due to a problem in squalane's absorption into skin.

To improve intended uses of squalane known as a new beauty material for beauty purposes, the inventors of the present invention turned their attention to fullerene with a special carbon structure, which are known as a material with high functionality. By combining squalane and fullerene, the inventors aimed at bringing out further health promotion and beauty effects.

### Disclosure of Invention

The object of the present invention is to develop a product with high functionality by utilizing carbon or fullerene and squalane together as raw materials and the synergistic effect of these raw materials.

Additionally, the object of the present invention is to develop a method for producing a complex material possessing the functionality of carbon or fullerene and squalane.

Furthermore, by industrial applicability of a product with high functionality, which is obtained using carbon or fullerene and squalane as raw materials, particularly by its intended uses for health promotion and beauty purposes, the object of the present invention is to develop a beauty product based on the raw materials' skin care effects, a food preservative, a food freshness preserving agent based on their antifungal effect, an insect repellent based on their effect in repelling harmful insects, a deodorant based on their fragrant odor effect or a baldness remedy based on their effect in promoting hair growth.

The basic characteristic of the present invention is that squalane containing ultrafine particles of carbon combustion residue containing a minute amount of fullerene is produced by combusting mixed gas of oxygen and hydrogen in squalane in a vacuum and by heating and combusting carbon with the combustion gas obtained. With the method possessing this characteristic, the above-mentioned objects are to be solved.

In other words, the present invention is basically presented by (1) to (7) described as follows:
(1) A health promotion material using squalane containing ultrafine particles of carbon combustion residue.
(2) The health promotion material mentioned in (1) above, which contains fullerene as ultrafine particles of carbon combustion residue.
(3) A method for producing squalane containing ultrafine particles of carbon combustion residue by combusting mixed gas of oxygen and hydrogen in squalane in a vacuum and by heating and combusting a carbon material with the combustion gas obtained.
(4) A method for producing squalane containing ultrafine particles of the above-mentioned carbon combustion residue, which is characterized in that the ultrafine particles of the above-mentioned carbon combustion residue are fullerene floating in squalane.
(5) A device for producing squalane containing ultrafine particles of the above-mentioned carbon combustion residue, which comprises a vacuum tank housing squalane, a spray nozzle of mixed gas of oxygen and hydrogen, a carbon feeding unit, an igniter, and a combustion chamber.
(6) A device for producing squalane containing ultrafine particles of the above-mentioned carbon combustion residue, to which a water-electrolyzer for producing oxygen and hydrogen is attached.
(7) The health promotion material mentioned in (1) above, which is characterized in that the material is a beauty product based on the material's skin care effects, a food preservative, a food freshness preserving agent based on its antifungal effect, an insect repellent based on its effect in repelling harmful insects, a deodorant based on its fragrant odor effect, or a baldness remedy based on its effect in promoting hair growth.

### [Brief Description of the Figures]

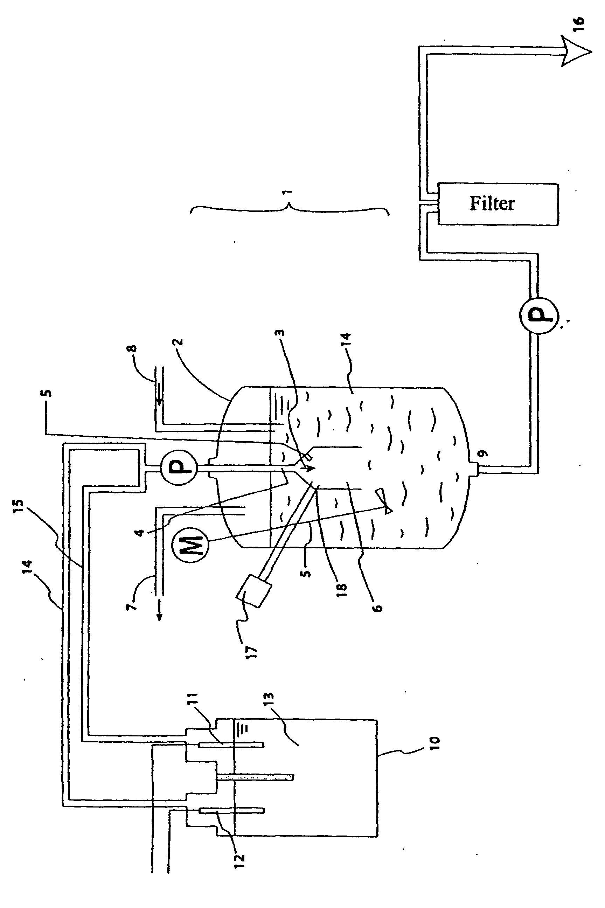

FIG. 1: A device for producing squalane containing fullerene according to the present invention

### [Explanation of symbols used]

- 1: Vacuum container
- 2: Vacuum tank
- **3**: Complete combustion area
- 4: Mixed gas spray nozzle
- 5: Stirrer
- 6: Combustion chamber
- 7: Vacuum path
- 8: Squalane supply path
- 9: Discharge path
- 10: Water-electrolyzer
- 11: Electrode
- 12: Electrode
- 13: Water
- 14: Hydrogen
- 15: Oxygen
- 16: Squalane recovery system
- 17: Carbon rod feed unit
- 18: Carbon rod
- 19: Squalane
- 20: Igniter

### Best Modes for Carrying out the Invention

The best mode for carrying out the Invention is described specifically based on an embodiment. The best mode for carrying out the Invention, however, is not limited to the described mode here.

Fig. 1 shows a device 1 for producing squalane containing ultrafine particles of carbon combustion residue such as fullerene.

Hydrogen 14 and oxygen 15, which are generated by electrolyzing water 13 housed in a water-electrolyzer 10 (11 and 12 are electrodes.), are supplied to a combustion chamber 6 in a vacuum tank 2 for producing squalane by controlling a flow by a pump and using a mixed-gas spray nozzle 4. Squalane is supplied into the vacuum tank 2 possessing a liquid stirrer 5 in advance from a feed path 8. The combustion chamber 6 possesses a carbon rod 18 which is supplied by a carbon rod feed unit 17. Inside the combustion chamber 6, contacting the carbon rod 18, which was ignited by an igniter 20 and is combusting, the mixed gas combusts fiercely in a complete combustion area 3. After combustion is completed, carbon combustion residue disperses in squalane aqueous dispersion 19, and squalane in which a minute amount of carbon combustion residue disperses is obtained.

The vacuum tank 2 is a main portion of a device 1 for producing squalane and comprises a pressure-resistant tank made of metal, preferably steel. Squalane, a raw material, is supplied to the vacuum tank 2 from squalane supply path 8. The vacuum tank 2 is kept nearly at a vacuum by a vacuum pump (not shown) through a vacuum path 7.

Mixed gas of oxygen and hydrogen is supplied to the combustion chamber 6 from the mixed-gas spray nozzle 4 and is combusted in the complete combustion area 3. The mixed gas is supplied from respective gas cylinders. Additionally, preferably by attaching a water-electrolyzer 10 for producing mixed gas of oxygen and hydrogen, the mixed gas is supplied directly by electrolyzing water. A carbon raw material is supplied as a carbon rod or a graphite rod by the carbon rod feed unit 17, and is gradually delivered into the combustion chamber 6 according to an amount already combusted. A position to insert a carbon rod should be an area in which mixed gas has combusted completely and vaporized completely into ultra-high temperature water vapor. Setting the complete combustion area 3 is extremely important. Inside the combustion chamber, the mixed gas is ignited by the igniter 20 and combusts, and ultrafine particles of carbon combustion residue are generated in squalane. At this time, a minute amount of fullerene is generated. Squalane containing carbon combustion particles containing a minute amount of fullerene, which was produced by combusting the mixed gas of oxygen and hydrogen in squalane by a carbon rod, is recovered from the discharge path of the vacuum tank into a squalane recovery system 16 after going through filtration equipment.

One example of working conditions of a device producing squalane containing the above-mentioned fullerene is as shown below.

### Working Conditions (Example)

Degree of vacuum of a vacuum tank: 100mmHg
Mixed gas supplied: 2 L/sec. (2 atm)
Spray time: 0.5 hr.
Squalane supplied: 100 L
Carbon supplied: 0.3kg/0.5 hr.
Squalane generated: 100 L

As mentioned above, raw material squalane is squalane extracted from liver oil obtained from deep sea shark extracts, getting denatured and being refined by a means such as adding hydrogen. Raw material carbon used in the present invention is supplied as a carbon rod or a graphite rod. Carbon is supplied as pure carbon form as possible so that no impurities are contained in a product.

In the present invention, mixed gas preferably of oxygen and hydrogen is used. The reason for this is not to generate any substances other than water and ultrafine particles of carbon residue and to prevent igniting and exploding of squalane. For these reasons, a position to putting in carbon should be an area in which mixed gas has combusted completely and vaporized completely into ultra-high temperature water vapor. A mixing ratio of oxygen and hydrogen should be controlled strictly to be one to two for achieving complete combustion. For that purpose, obtaining respective raw material gases by electrolyzing water is preferable because the one-two mixing ratio is maintained without particularly controlling a gas ratio. Using of a vacuum tank is to prevent igniting of squalane. A degree of vacuum is set at approximately 100 mmHg.

At the time of combustion in the device according to the present invention, due to impulse wave generated at the combustion portion, and combustion, squalane is emulsified with squalane as combustion residue and water generated. As this emulsion will be separated into water and squalane after passage of a bit of time, supernatant squalane should be collected so that squalane is not mixed with precipitated water. Alternatively, a squalane layer can be recovered after discharging precipitated water from an exhaust path.

Ultrafine particles of carbon combustion residue having extremely strong hydrophobicity, which are obtained by the present invention, float stably in squalane without mixing with water, and the particles appear to be dissolving to the eye.

In these processes, if an amount of the ultrafine particles of carbon combustion residue to be generated in squalane increases, the appearance of squalane may be tinged with black color. This black color can become a problem depending on intended uses, thus it is necessary to control reaction time and an amount of heat applied (a combustion amount of fuel gas). If the time and the amount of heat are not sufficient, an amount to be generated of ultrafine particles of carbon combustion residue is not sufficient. Consequently, the functionality of squalane obtained is inferior. If the amount is excessive, squalane produced becomes blackish and cannot be used depending on its intended uses. In this case, if 100 L of squalane is to be produced, a spray amount of gas is preferably approximately 2 L per second and spray time is preferably approximately 30 minutes.

In the device according to the present invention for producing squalane, when carbon heated at high temperature inside the combustion chamber is discharged in squalane, the carbon discharged changes to ultrafine particles and the particles float in squalane. The size of the particle is chiefly from several microns to tens microns. At this time, a minute amount of fullerene is generated. In other words, when fullerene is generated, a part of carbon takes a crystal structure as mentioned before. This crystal structure is thought to be a morning-glory-flower-shape like a carbon structure consisting of five hexagon structures called "corannulene". Carbon atoms, then, are rearranged, changing to a nearly spherical shape, and fullerene having a cage structure larger than a water molecule changes to a soccer-ball shape, which is an energy-wise stable structure. Fullerene is a carbon material; typically it has a spherical structure comprising 60 carbon atoms; it is a molecule having a structure of a soccer-ball-like pattern combining pentagons and hexagons; it is a physically and chemically stable molecule. This device generates fullerene in a molecular condition of an ultrafine particle on a nanomicron scale, which is larger than a water molecule. Although it is not known that fullerene displays a variety of high functionality because of its specific molecular structure or ultrafine particle condition, it is thought to possess effects to maintain human body health and to treat human being.

The significant synergistic effect for health, beauty and treatment functions of a complex material comprising ultrafine particles of carbon combustion residue containing a minute amount of fullerene and squalane, which are obtained in the present invention is manifested in the embodiment described below and from physical property test findings of panty hose manufactured from fibers to which squalane containing ultrafine particles of carbon combustion residue is accreted.

Additionally, prospective various intended uses of squalane containing ultrafine particles of carbon combustion residue other than the above-mentioned include beauty products based on its skin care effects, food preservatives, food freshness preserving agents based on its antifungal effect, insect repellents based on its effect in repelling harmful insects, deodorants based on its fragrant odor effect or baldness remedies based on its effect in promoting hair growth.

### Test Examples

### Health promotion effect of squalane containing ultrafine particles of carbon combustion residue according to the present invention

By accreting squalane containing fullerene according to the present invention to a polyamide fiber (squalane 1g/fiber 100g) and manufacturing panty hose using the polyamide fibers, health promotion effect was tested by 20 monitors (females of 15 to 58 years of age). The length that monitors were asked to have the panty hose on was approximately two weeks.

For a comparative example, panty hose was manufactured with a process using only squalane.

### Test Findings

| | Embodiment | Comparative Example |
|---|---|---|
| Effect Example | | |
| Improvement of blood circulation | 12 persons | 2 persons |
| Treating of sensitivity to cold | 12 | 2 |
| Relieving of swollen feet | 11 | 7 |
| Reducing of weary feet | 18 | 3 |
| Relieving of athlete's foot | 9 | 1 |
| Treating of varices of foot | 2 | 0 |
| Treating of neuralgia | 3 | 0 |
| Comfortable foot | 20 | 7 |
| Recovery of rough dry skin on the foot | 8 | 6 |
| Recovery of fatigue of entire body | 16 | 2 |
| Improvement of anemia | 7 | 3 |
| Relieving of gout | 1 | 0 |

According to the above-mentioned test findings, foot-related health problems of women were relieved and medical conditions were treated. The findings show that squalane containing ultrafine particles of carbon combustion residue has remarkably excellent effects in health promotion and is a breakthrough product.

### Industrial Applicability

Utilizing carbon or fullerene and squalane, which are known as materials with high functionality and using them together, the present invention increases functionality by the synergistic effect of the materials, develops new functions and provides a new complex material responding to social needs including health consciousness.

Squalane containing fullerene, the new complex material obtained in the present invention, achieves remarkable effects on health promotion and treatment.

Additionally, the above-mentioned squalane comes in useful for such intended uses as beauty products having skin care effects, food preservatives, food freshness preserving agents having antifungal effect, insect repellents having the effect of repelling harmful insects, deodorants having a fragrant odor effect or baldness remedies having the effect of promoting hair growth.

## Claims

1. A health promotion material, which is **characterized by** comprising squalane containing ultrafine particles of carbon combustion residue.

2. The health promotion material as claimed in Claim 1, which is **characterized in that** the ultrafine particles of carbon combustion residue are fullerene.

3. A method for producing squalane containing ultrafine particles of carbon combustion residue, which is **characterized in that** mixed gas of oxygen and hydrogen is combusted in squalane in a vacuum and then a carbon is heated' and combusted with the combustion gas obtained.

4. The method for producing squalane as claimed in Claim 3, which is **characterized in that** the ultrafine particles of carbon combustion residue are fullerene floating in squalane.

5. A device for producing squalane containing ultrafine particles of carbon combustion residue, which is **characterized by** comprising a vacuum tank housing squalane, a spray nozzle for mixed gas of oxygen and hydrogen, a carbon feeding unit, an igniter, and a combustion chamber.

6. The device for producing squalane as claimed in Claim 5, which is **characterized by** further comprising a water-electrolyzer for producing mixed gas of oxygen and hydrogen.

7. The health promotion material as claimed in Claim 1, which is **characterized in that** said material is a beauty product, a food preservative, an agent for preserving freshness of foods, an insect repellent, or a deodorant.
